# EUROPEAN PATENT SPECIFICATION

(11) **EP 3 030 551 B1**
(45) Date of publication and mention of the grant of the patent: **27.11.2019**
(21) Application number: 14750307.2
(22) Date of filing: 30.07.2014
(51) Int. Cl.: C07D 277/56

(54) **A NOVEL PROCESS FOR THE PREPARATION OF FEBUXOSTAT**
NEUARTIGES VERFAHREN ZUR HERSTELLUNG VON FEBUXOSTAT
NOUVEAU PROCÉDÉ DE PRÉPARATION DE FEBUXOSTAT

(30) Priority: 07.08.2013 WO PCT/EP2013/002361
(43) Date of publication of application: 15.06.2016
(73) Proprietor: Pharmathen S.A., 15351 Pallini Attikis (GR)
(72) Inventor: KOFTIS, Theocharis, V., GR-57001 Thessaloniki (GR); NEOKOSMIDIS, Efstratios, GR-57001 Thessaloniki (GR); TRAKOSSAS, Sakellarios, GR-57001 Thessaloniki (GR); PANAGIOTIDIS, Theodoros, GR-57001 Thessaloniki (GR); ANDREOU, Thanos, GR-57001 Thessaloniki (GR); VARVOGLI, Anastasia-Aikaterini, GR-57001 Thessaloniki (GR)
(86) International application number: PCT/EP2014/002079
(87) International publication number: WO 2015/018507

(56) References cited:
- WO-A1-2005/095400
- WO-A1-2012/066561
- WO-A2-2011/141933
- CN-B- 101 412 700
- TALUKDAR S ET AL: "Direct transformation of aldehydes to nitriles using iodine in ammonia water", TETRAHEDRON LETTERS, PERGAMON, GB, vol. 42, no. 6, 5 February 2001 (2001-02-05), pages 1103-1105, XP004316696, ISSN: 0040-4039, DOI: 10.1016/S0040-4039(00)02195-X
- CHUANZHOU TAO ET AL: "Copper-catalyzed aerobic oxidative synthesis of aryl nitriles from benzylic alcohols and aqueous ammonia", ORGANIC & BIOMOLECULAR CHEMISTRY, vol. 11, no. 20, 20 March 2013 (2013-03-20), page 3349, XP055138895, ISSN: 1477-0520, DOI: 10.1039/c3ob00002h

## Description

### TECHNICAL FIELD OF THE INVENTION

The present invention relates to a novel preparation method for 2-(3-cyano-4-isobutoxyphenyl)-4-methyl-1,3-thiazole-5-carboxylic acid (Febuxostat) via novel and high yielded conversion of a formyl group into a cyano group.

### BACKGROUND OF THE INVENTION

Febuxostat (Formula I) is an inhibitor of xanthine oxidase, which was discovered by the Japanese company Teijin Pharma Ltd and it is indicated for use in the treatment of hyperuricemia and chronic gout. Its chemical name is 2-(3-cyano-4-isobutoxyphenyl)-4-methyl-1,3-thiazole-5-carboxylic acid. It is marketed under the brand names Adenuric in Europe, Feburic in Japan and Uloric in USA and Canada.

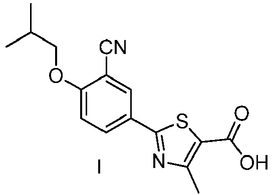

In EP0513379B1 Febuxostat is prepared from 4-hydroxy-3-nitrobenzaldehyde, according to the following scheme.

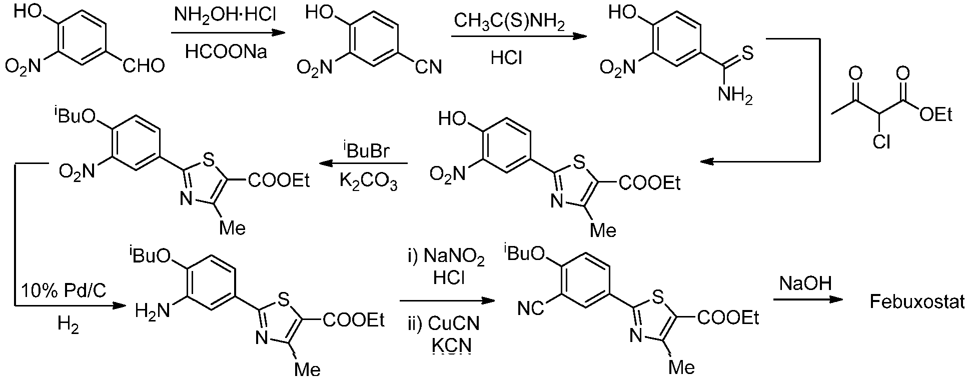

This particular process suffers from major drawbacks. Not only it is very long, including seven steps from the starting material to the final product, but, most importantly, it employs the use of cyanides, which are extremely toxic reagents. Cyanide salts are likely to generate hydrocyanide, which sets a high amount of risk in an industrial scale process.

In Japanese patent JP06345724A (JP2706037B) the intermediate ethyl ester of Febuxostat is prepared from p-cyano-nitrobenzene, in three steps. Febuxostat may, then, be prepared by alkaline hydrolysis, according to prior art.

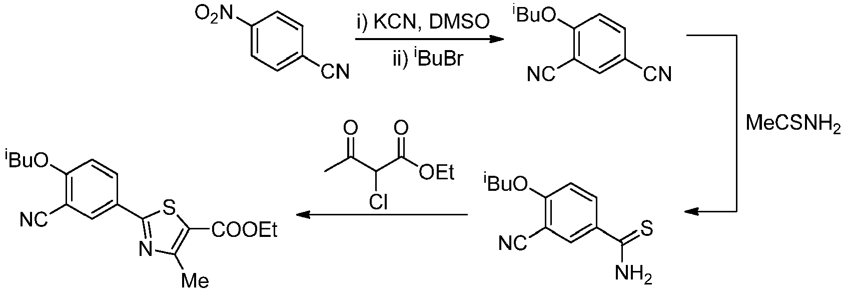

The use of extremely toxic potassium cyanide makes this process unsuitable for manufacturing purposes.

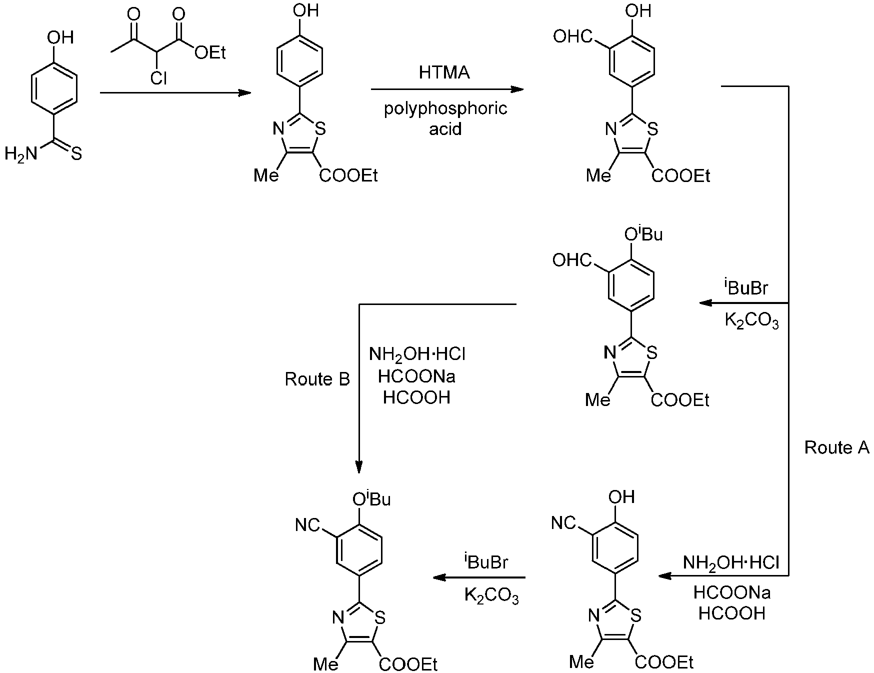

In Japanese patent JP3202607B Febuxostat ethyl ester is prepared, according to the above scheme, through two similar routes. Route A uses flash column chromatography for the purification of the hydroxylamine reaction product, while Route B suffers from low yield and the use of chlorinated solvents for recrystallization. In addition, the reaction solvent is, in both cases, formic acid which causes severe skin burns and eye damage to humans. Formic acid is also corrosive towards metal-based materials of construction (MOC), like stainless steel and nickel alloys, limiting the options, essentially, to glass reactors or vessels. The drawbacks of using this solvent are also related to the high volumes of formic acid required per batch, which hinder the waste treatment.

In a similar manner, according to example 11 in WO2011141933, the hydroxylamine reaction utilizes formic acid in high proportion and temperature, rendering the process problematic in large scale, in terms of equipment selection, safety and waste management.

In CN101723915B focus is made to the improvement of the hydroxylamine reaction. Formic acid is replaced with dimethylformamide (DMF) and other solvents. However, according to widely used organic chemistry textbooks, such as March's Advanced Organic Chemistry, p1287, 6th edition, M. B. Smith and J. March, ISBN 0-471-72091-7, the mechanism of the reaction involves the formation of an oxime, upon the action of hydroxylamine, which further dehydrates to form a nitrile, with the aid of a suitable reagent, for example formic acid, or acetic anhydride. In the absence of such a reagent, it is expected that the reaction will, at least, not lead to completion, thereby leading to low yields and undesired impurity levels, namely the intermediate oxime. Such impurities, arising from the reactions of the process and which exhibit similar structure of the desired product, are often difficult to remove with common industrial techniques, e.g. crystallization.

In a similar manner, WO2012066561 performs the hydroxylamine reaction in polar aprotic solvents, preferably DMF solvent, with addition of acetyl chloride, thus avoiding use of formic acid.

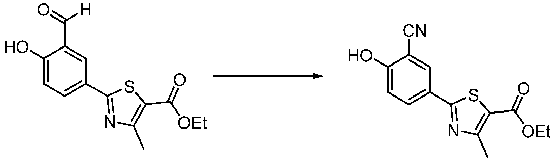

In WO2010142653A1 the intermediate Febuxostat ethyl ester is prepared from 4-cyanophenol, through a five-step process. Febuxostat can be prepared from its respective ethyl ester via alkaline hydrolysis, as in the previous case.

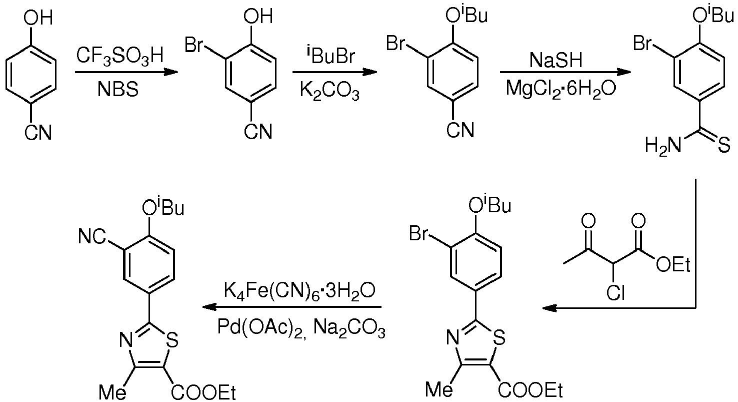

The process employs, in the final step, the use of palladium catalyst and, moreover, the reaction is performed at elevated temperatures (145 °C) for 48 hours, conditions that raise the energy cost and are, in general, difficult to transfer in industrial scale.

WO2005095400 discloses a method for transforming an ortho-substituted aromatic methoxy-formyl compound to the corresponding nitrile analogue, using molecular iodine and ammonia. The same methodology was disclosed by Talukdar et al. in Tetrahedron Letters, 42, 6, 2001, pp 1103-1105 for aromatic, heterocyclic, aliphatic, conjugated and polyhydroxy aldehydes.

A similar transformation to the key reaction of the present invention, i.e. oxidation of aldehydes to nitriles is disclosed by Tao et al., in Organic and Biomolecular Chemistry, 11, 20, 2013, p 3349, although the primary object of their study was the aerobic oxidative synthesis of aryl nitriles from the corresponding benzylic alcohols. The authors evaluated the transformation of aryl aldehydes to aryl nitriles using Cu(NO₃)₂/NH₃/O₂ and concluded that the yields were highly dependent on the nature of the substitution of the aryl group, in terms of their electron-withdrawing ability and ranged from a low 5% to as high as 89%. No experimental evidence or even reference is given though that the same reaction conditions could be applied to achieve - in yields acceptable in industrial scale manufacture - the transformation of aromatic aldehydes bearing an ester group as will be shown in the present invention. Accordingly, there is still a need for a process suitable to produce Febuxostat compound in higher yields, exhibiting a chemical purity that meets national or international authorities' standards, with an industrially viable, convenient and safe method.

### SUMMARY OF THE INVENTION

The invention provides a process for the conversion of a formyl group of compound of formula II into a cyano group of compound of formula III, wherein R₁ is a hydrogen atom, an alkyl, alkenyl, or alkynyl group and R₂ is an alkyl, alkenyl, or alkynyl group, in the presence of ammonia and oxygen and metal catalyst, wherein the temperature range is 60-120°C.

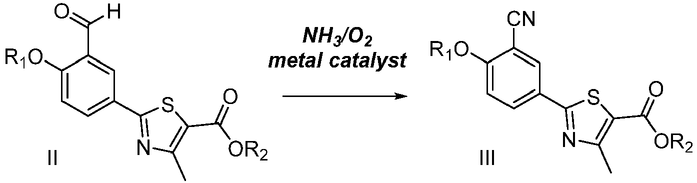

Another object of this invention is to provide a novel process for the preparation of Febuxostat, exhibiting improved yield, safe reagents and industrially applicable techniques.

A further object of the invention is a process for the production of Febuxostat, comprising the following steps:
a) alkylation of compound of formula IIa where R₁ is a hydrogen atom and R₂ is an alkyl, alkenyl, or alkynyl group, preferably an ethyl group to form a compound of formula IIb where R₁ is *iso*-butyl and R₂ is an alkyl, alkenyl, or alkynyl group, preferably an ethyl group; 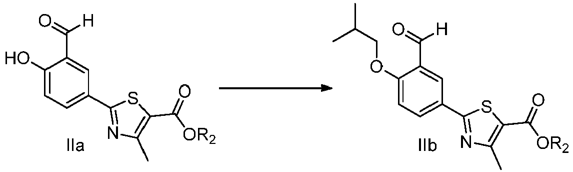
b) conversion of the formyl group of compound of formula IIb to cyano group, to produce compound of formula IIIb, in the presence of: ammonia, oxygen, and a metal catalyst wherein the temperature range is 60-120 °C; 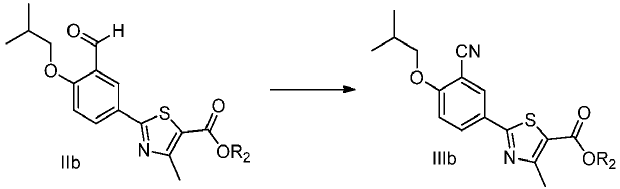
c) hydrolysis of the ester group of compound of formula IIIb to produce Febuxostat, or a salt thereof;

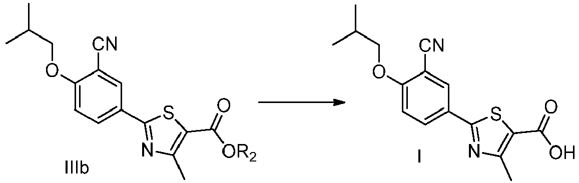

We have found that the route above will also perform as well when the steps a) and b) are reversed. Therefore, it is another object of the present invention, to provide an alternate route for the production of Febuxostat, comprising:
a) conversion of formyl group of compound of formula IIa where R₁ is a hydrogen atom and R₂ is an alkyl, alkenyl, or alkynyl group, preferably an ethyl group to cyano group, to produce compound of formula IIIa where R₁ is a hydrogen atom and R₂ is an alkyl, alkenyl, or alkynyl group, preferably an ethyl group, in the presence of ammonia, oxygen, and a metal catalyst; 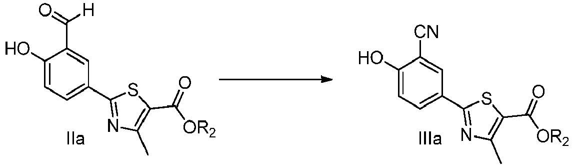
b) alkylation of compound of formula IIIa where R₁ is a hydrogen atom and R₂ is an alkyl, alkenyl, or alkynyl group, preferably an ethyl group to compound of formula IIIb where R₁ is *iso*-butyl and R₂ is an alkyl, alkenyl, or alkynyl group, preferably an ethyl group; 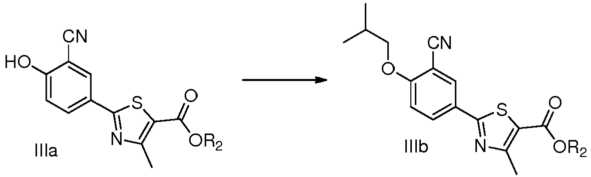
c) hydrolysis of the ester group of compound of formula IIIb to produce Febuxostat, or a salt thereof.

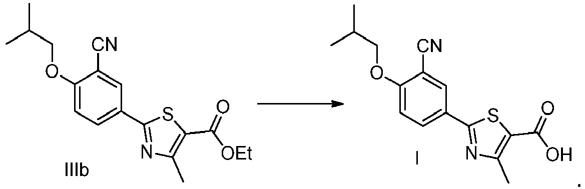

According to the present invention, the transformation of the formyl group to cyano group in a compound of formula II, in either of the processes described above, is performed in the presence of ammonia, oxygen, and a metal catalyst wherein the temperature range is 60-120 °C.

Another object of the present invention is a process, for the preparation of Febuxostat, comprising the conversion of the formyl group of compound of general formula II, to cyano group of compound of formula III, in the presence of ammonia, oxygen and a metal catalyst wherein the temperature range is 60-120 °C, and subsequent conversion of the compound of general formula III to Febuxostat. Conversion of the compound of general formula III to Febuxostat is readily achieved when R₂ is other than hydrogen by removing the alkyl, alkenyl, or alkynyl group and, where R₁ is other than *iso*-butyl, converting R₁ to *iso*-butyl.

### DETAILED DESCRIPTION OF THE INVENTION

In the present invention a novel pathway for the conversion, of the formyl group of compound of formula II into the cyano group of compound of formula III, is demonstrated, where R₁ is a hydrogen atom, an alkyl, alkenyl, or alkynyl group and R₂ is an alkyl, alkenyl, or alkynyl group, preferably an ethyl group in the presence of ammonia, oxygen and metal catalyst, wherein the temperature range is 60-120°C.

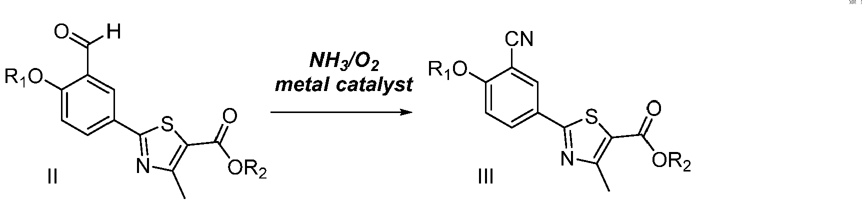

The present invention also encompasses the preparation of Febuxostat or salts thereof, through this novel transformation, included in the process in the below scheme. The process is advantageous over prior art methods, due to the fact that it features high reaction yields, leads to compounds with chemical purities suitable for pharmaceutical purposes, uses more safe reagents and possesses characteristics suitable for industrialization.

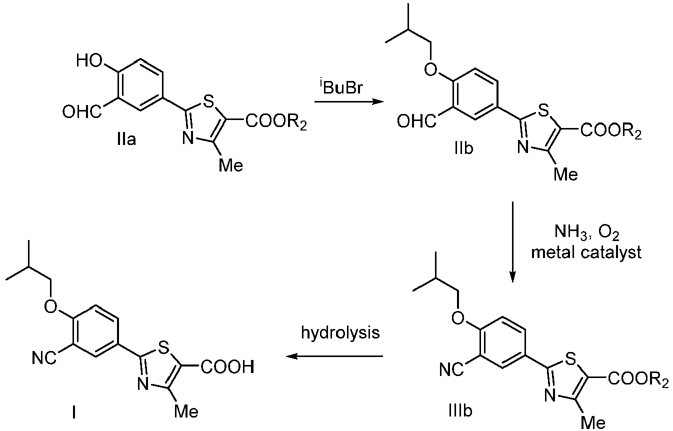

A particular object of the invention is to provide a process for the preparation of Febuxostat, comprising the following steps:
a) alkylation of compound of formula IIa where R₁ is a hydrogen atom and R₂ is an alkyl, alkenyl, or alkynyl group, preferably an ethyl group to compound of formula IIb where R₁ is an *iso*-butyl group and R₂ is an alkyl, alkenyl, or alkynyl group, preferably an ethyl group; 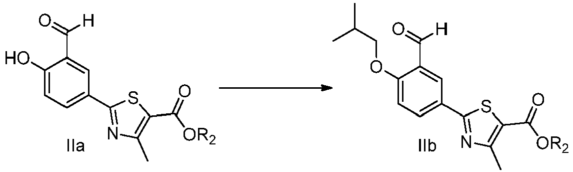
b) conversion of the formyl group of compound of formula IIb to cyano group, to produce compound of formula IIIb, in the presence of ammonia, oxygen and a metal catalyst, wherein the temperature range is 60-120 °C; 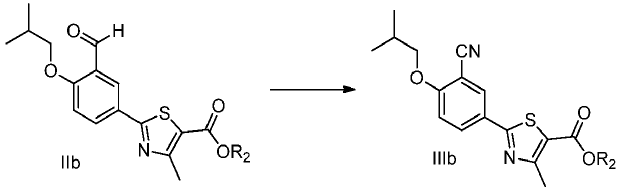
c) hydrolysis of the ester group of compound of formula IIIb to produce Febuxostat, or a salt thereof.

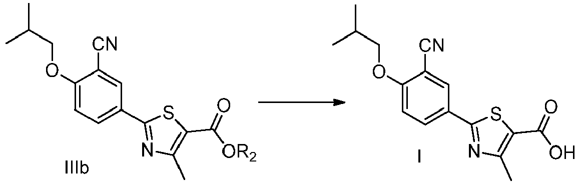

Suitable ester groups for preparation of Febuxostat are methyl, ethyl, propyl, *iso*propyl, butyl, *iso*-butyl, *tert*-butyl preferably an ethyl ester group.

In step a, the etherification reaction is performed with an isobutyl halide, preferably isobutyl bromide, in the presence of a base. The base can be an inorganic base. Preferable inorganic bases are metal hydroxides and carbonates. More preferable inorganic bases are potassium carbonate, sodium carbonate, lithium carbonate. The base may also be an organic base. Preferable organic bases are amines. More preferable organic bases are trimethylamine, triethylamine, diisopropylamine, diisopropylethylamine, *N,N*-dimethylaminopyridine. The reaction is performed at temperatures that range between 25-100 °C. Preferable temperatures are 50-80 °C. Solvents suitable for the reaction are polar aprotic solvents. Preferable polar aprotic solvents are dimethylsulfoxide, dimethylformamide, dimethylacetamide, *N-*methylpyrrolidone, tetrahydrofuran, acetonitrile, acetone, *t*-butyl methyl ether.

In step b, the conversion of the formyl group to cyano group is performed with ammonia, an oxygen source and a metal catalyst. As a metal catalyst various compounds of metals may be used. Non-limiting examples of metals are copper, iron, zinc, tin, ruthenium, palladium, rhodium, iridium, silver, cobalt, nickel, manganese, molybenium, vanadium and rhenium. Preferred metals are copper, iron, ruthenium, palladium, iridium and silver. More preferred metals are copper, iron and ruthenium. Non-limiting examples of metal catalysts are oxides, hydroxides, salts of metals with the conjugated base of either strong acids, such as hydrohalides, sulfuric acid, nitric acid, or organic acids, such as triflic acid and acetic acid.

The amount of ammonia used at the reaction may well vary, depending on the scale of the reaction and the conditions employed to it. Normally, at reactions involving such volatile reagents, the latter are used in great excess. Moreover, the amount of ammonia used in the reaction depends on the form in which the reagent is available. Non-limiting examples are aqueous solutions of ammonia and gaseous ammonia. The solvent can be a typical organic solvent. Preferable organic solvents are polar aprotic solvents. Preferable polar aprotic solvents are dimethylsulfoxide, dimethylformamide, dimethylacetamide, *N*-methylpyrrolidone, tetrahydrofuran, acetonitrile, acetone, *t-*butylmethylether. The temperature, at which the reaction is performed, may range from room temperature to the boiling point of the respective solvent.

In step c, the hydrolysis of the ester may be performed under basic conditions. Such conditions involve a strong inorganic base. Preferable bases are metal oxides, hydroxides and carbonates. More preferable bases are alkali metal and alkaline earth oxides, hydroxides and carbonates. More preferable bases are sodium hydroxide, potassium hydroxide, lithium hydroxide, barium hydroxide, barium oxide, sodium carbonate, potassium carbonate and lithium carbonate. The reaction may be performed in a variety of solvents, depending mainly on the nature of the base used at it. Typical organic solvents suitable for this reaction are polar protic and aprotic solvents, as well as mixtures of them with water. Preferred polar aprotic solvents are tetrahydrofuran, acetone, *t*-butyl methyl ether, ethyl acetate. Preferred polar protic solvents are lower alcohols. More preferable solvents are methanol, ethanol, n-propanol and *iso*-propanol. The reaction can be performed at room temperature to the boiling point of used solvent. Preferable temperature range is from 20 to 80 °C.

We have found that the route above will also perform as well when the steps a) and b) are performed in reverse sequence. The conditions and preferred conditions apply equally. Therefore, another object of the invention is to provide an alternate process for the preparation of Febuxostat, comprising the following steps:
a) conversion of formyl group of compound of formula IIa where R₁ is a hydrogen atom and R₂ is an alkyl, alkenyl, or alkynyl group, preferably an ethyl group to cyano group, to produce compound of formula IIIa where R₁ is a hydrogen atom and R₂ is an alkyl, alkenyl, or alkynyl group, preferably an ethyl group, in the presence of ammonia, oxygen, and a metal catalyst, wherein the temperature range is 60-120 °C;
b) alkylation of compound of formula IIIa where R₁ is a hydrogen atom and R₂ is an alkyl, alkenyl, or alkynyl group, preferably an ethyl group to compound of formula IIIb where R₁ is an *iso*-butyl group and R₂ is an alkyl, alkenyl, or alkynyl group, preferably an ethyl group;
c) hydrolysis of the ester group of compound of formula IIIb to produce Febuxostat, or a salt thereof.

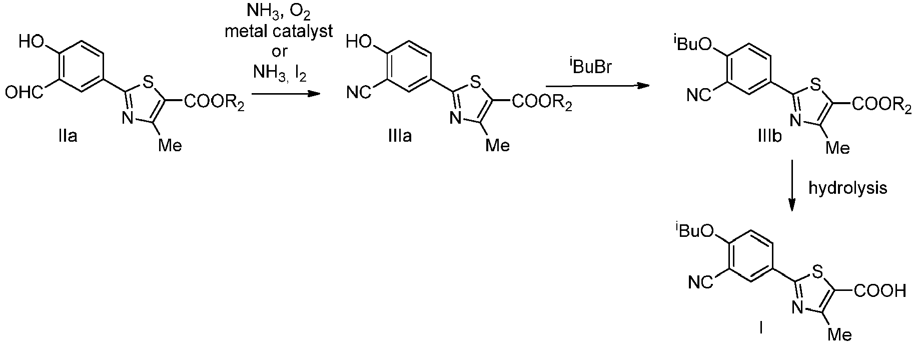

The first step in this sequence is only according to the invention when reacting the compound of formula IIa with ammonia, oxygen and the metal catalyst as defined under step a) prior to the scheme. Using ammonia and iodine is not according to the invention but for reference only. Preferred conditions for individual steps of the process are as already described above.

In yet another embodiment of the present invention, the metal catalyst used in step b is a copper catalyst, an iron catalyst, or a ruthenium catalyst.

In still another embodiment of the invention, the metal catalyst is a copper catalyst. The copper catalyst may be selected from inorganic compounds and salts of copper, of oxidative state (I) or (II). Preferable compounds and salts are copper halides, copper nitrate, copper acetate, copper sulfate, copper triflate, copper oxide and their hydrates.

In the present invention, the conversion of the formyl group of formula II to cyano group of formula III is carried out in the presence of ammonia, an oxygen source and a metal catalyst.

In a preferred embodiment of the present invention, the above described conversion is carried out in a polar aprotic solvent. Preferred aprotic solvents are dimethylsulfoxide, dimethylformamide, dimethylacetamide, *N*-methyl pyrrolidone and tetrahydrofuran.

In the present invention, the above described conversion of the formyl group of formula II to cyano group of formula III is carried out in temperatures ranging from 60-120 °C. Preferably, the temperature range is 70-110 °C.

In still another embodiment of the invention, said conversion of the formyl group of formula II to cyano group of formula III is performed under oxygen atmosphere. The percentage of the oxygen which is present in the reaction atmosphere may range from 1% to 100%. Preferable range is 5% to 100%. More preferable range is 20% to 100%. It will be acknowledged, from the person skilled in the art, that when the atmosphere of the reaction is less than 100%, the remaining percentage may express other gases. Non-limiting examples are nitrogen, noble gases, methane, hydrogen and carbon dioxide. Therefore, the definition may be interpreted as including the composition of atmospheric air as well.

It will further be acknowledged, from the person skilled in the art that the reaction time may vary depending on the percentage of oxygen present in the air supply, used in the reaction. The reaction time may also vary, depending on the pressure developed or applied to the reaction.

Another object of the present invention is a process for the preparation of Febuxostat, as described above, comprising the conversion of the formyl group of compound of formula II, to nitrile group of compound of formula III, comprising ammonia, oxygen, and a metal catalyst, wherein the temperature range is 60-120 °C and subsequently converting the compound of formula III to Febuxostat.

### EXPERIMENTAL

### EXAMPLE 1: Preparation of compound of formula IIb

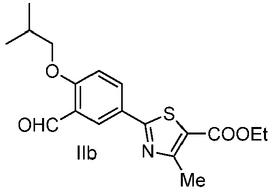

Dissolve 14.14g of ethyl 2-(3-formyl-4-hydroxyphenyl)-4-methylthiazole-5-carboxylate (Formula III) in 55 ml dimethylformamide, at ambient temperature. Add 40g of potassium carbonate, along with 15.9 ml isobutyl bromide. Heat the reaction to 75-80 °C and stir for 4 hours. Cool to 25-30 °C, while 165 ml process water is added. Further cool to 0-5 °C and stir for 30 minutes at this temperature. Filter off the precipitated solid and wash the filter cake with 55 ml process water. The wet cake is dried under vacuum at 40 °C for 7 hours, to furnish 16.43 g of ethyl 2-(3-formyl-4-isobutoxyphenyl)-4-methylthiazole-5-carboxylate (Formula IIb).

### EXAMPLE 2: Preparation of compound of formula IIIb

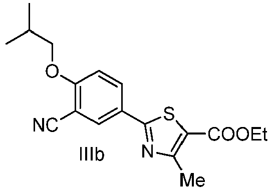

In a 25 mL round-bottomed flask charge under stirring at 25-30 °C, 1.0 g (2.88 mmol) of ethyl 2-(3-formyl-4-isobutoxyphenyl)-4-methylthiazole-5-carboxylate in 3.0 mL dimethylformamide. Add 34 mg (0.19 mmol) copper acetate under stirring at 25-30 °C. Flush with oxygen (O₂) and add 0.66 ml (34.92 mmol) 25% aqueous ammonia. Flush again with O₂. Heat the reaction mixture to 80-82 °C overnight. Check the progress of the reaction by TLC (cyclohexane:ethyl acetate 3:1). Cool reaction mass to 25-30 °C. Add 25mL ethyl acetate and 25mL brine at the reaction mass, separate organic layer and extract aqueous layer twice with 25mL ethyl acetate. Combine organic layers, dry over anhydrous sodium sulfate, filter off and concentrate till dry. The residue is purified with column chromatography (cyclohexane:ethyl acetate 9:1). afforded 0.754g of ethyl 2-(3-cyano-4-isobutoxyphenyl)-4-methylthiazole-5-carboxylate (Formula IIIb) Yield: 75.4%.

### EXAMPLE 3 (for reference only): Preparation of compound of formula IIIb

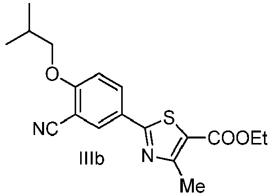

In a 25 mL round-bottomed flask charge under stirring at 25-30 °C, 0.17 g (0.49 mmol) of ethyl 2-(3-formyl-4-isobutoxyphenyl)-4-methylthiazole-5-carboxylate in 2.5 mL tetrahydrofuran. Add 2.9 mL (153.43 mmol) 25% aqueous ammonia, under stirring at 25-30 °C. Add 137 mg (0.54 mmol) iodine (I₂) to the reaction mass, stir the reaction mixture at 25-30 °C for 15-30 min. Check the progress of the reaction by TLC (cyclohexane:ethyl acetate 3:1). Starting material is consumed. Add 2.5 mL 5% w/v aqueous sodium thiosulfate Na₂S₂O₃ and 15mL ethyl acetate at the reaction mass, separate organic layer and extract twice aqueous layer with 15mL ethyl acetate. Combine organic layers, dry over anhydrous sodium sulfate, filter off and concentrate till dry. 0.158g of ethyl 2-(3-cyano-4-isobutoxyphenyl)-4-methylthiazole-5-carboxylate (Formula IIIb) are collected.

### EXAMPLE 4: Preparation of Febuxostat

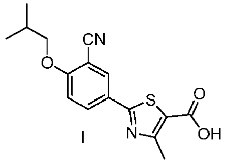

In a 100 ml 2-neck round-bottomed flask charge 2.407g of ethyl-2-(3-cyano-4-isobutoxyphenyl)-4-methylhiazole-carboxylate in 20ml tetrahydrofuran under stirring, at 25-35 °C, 0.748g of sodium hydroxide and heat reaction mass to 60-65 °C for approximately 8 hrs. Check the progress of the reaction by TLC (cyclohexane:ethyl acetate 3:1). Cool reaction mass to 0-5 °C and add 50 ml process water keeping temperature within 0-5 °C. Adjust pH to 1-2 with 4.5 ml 6 N hydrochloric acid, keeping temperature within 0-5 °C. Warm up reaction mass to 25-30 °C and stir reaction mass at the above temperature for 15 min. Filter off the precipitated solid through Buchner funnel under reduced pressure, spray wash with 2 ml process water and suck dry for 20-30 min. Transfer the crude solid in a 50 ml round-bottomed flask, charge 12 ml process water and 12 ml acetone at 25-30°C. Heat the reaction mass to 50-60 °C for 60 min. Cool down reaction mass to 0-5 °C and stir for 60 min at the above temperature. Filter off the precipitated solid though Buchner funnel under reduced pressure, spray wash with 2 ml of a 1:1 mixture of acetone and process water and suck dry for 30-45 min. Dry under vacuum at 60 °C. 1.821g of (compound I) Febuxostat are collected, Purity: 82.6%, Yield: 0.62w/w.

### EXAMPLE 5 (for reference only): Preparation of compound of formula IIIa

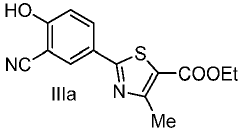

In a 50 mL round-bottomed flask charge under stirring 0.5g (1.72 mmol) of ethyl 2-(3-formyl-4-hydroxyphenyl)-4-methylthiazole-5-carboxylate in 8.6 mL THF, at 25-30 °C. Add 10.3 mL (544.94 mmol) 25% aqueous ammonia, under stirring at 25-30 °C. Add 480 mg (1.89 mmol) iodine (I₂) to the reaction mass, stir the reaction mixture at 25-30 °C for 15-30 min. Check the progress of the reaction by TLC (cyclohexane:ethyl acetate 1:1). Starting material is consumed. Add 8.6 mL 5% w/v aqueous thiosulfate and 40 mL ethyl acetate at the reaction mass, separate organic layer and extract aqueous layer twice with 40 mL ethyl acetate. Combine organic layers, dry over anhydrous sodium sulfate, filter off and concentrate to dryness. Purification of the residue with column chromatography (cyclohexane: ethyl acetate 3:1) afforded 0.213 g of ethyl 2-(3-cyano-4-hydroxyphenyl)-4-methylthiazole-5-carboxylate (Formula IIIa). Yield: 42.6%.

### EXAMPLE 6: Preparation of compound IIIb

Dissolve 2.2 g of ethyl 2-(3-cyano-4-hydroxyphenyl)-4-methylthiazole-5-carboxylate (Formula VI) in 7 ml dimethylformamide and to this mixture add 6.6 g of potassium carbonate and 3.14 g of isobutyl bromide. Stir the reaction at 75 °C for 15 hours and then cool to 40 °C. Add 15 ml process water and cool to 0-5 °C. Filter the precipitated solid off and wash with 15 ml process water, which, after drying, affords 2.28 g of ethyl 2-(3-cyano-4-isobutoxyphenyl)-4-methylthiazole-5-carboxylate (Formula IIIb).

### EXAMPLE 7: Preparation of compound I (Febuxostat)

In a 100 ml 2-neck round-bottomed flask charge 2.131 g of ethyl-2(3-cyano-4-isobutoxyphenyl)-4-methylhiazole-carboxylate, 64 ml methanol and 2.5 ml process water are added under stirring at 25-35 °C. Add 1.718 g potassium carbonate and heat reaction mass to reflux for approximately 2-3 hrs. Check the progress of the reaction by TLC (cyclohexane:ethyl acetate 3:1). Cool reaction mass to 20-25 °C. Concentrate solvent at below 40 °C. To the residue add 43 ml process water, 21 ml ethyl acetate and stir for 30 min at 25-35 °C. Separate layers and transfer aqueous layer in a 100 ml round-bottomed flask. Adjust pH to 2.3-2.7 with 25 ml 1 N hydrochloric acid, at 25-35 °C. Warm up reaction mass to 40 °C and stir reaction mass at this temperature for 60-90 min. Cool down reaction mass to 25-35 °C. Filter off the precipitated solid through Buchner funnel under reduced pressure, spray wash with 5 ml process water and suck dry for 30-45 min. Dry under vacuum at 60 °C. 1.708g of (compound I) Febuxostat are collected, Purity: 86.7%, Yield: 0.69w/w.

### EXAMPLE 8: Preparation of Febuxostat crystalline form III

In a 250 mL round-bottomed flask charge under stirring at 25-30 °C 10 g of crude 2-(3-cyano-4-isobutoxyphenyl)-4-methylthiazole-5-carboxylic acid (Febuxostat) in 200 mL ethyl acetate. Heat reaction mass to reflux and stir for 30 min. Cool reaction mass to 25-30°C. Warm again reaction mass and partially distill off solvent from the reaction mass at temperature below 40 °C under reduced pressure. Cool reaction mass to 25-30°C. Filter off the precipitated solid through Buchner funnel under reduced pressure and spray wash with 10 mL ethyl acetate. Dry under vacuum at 60°C. 8.5 g of Febuxostat are collected. Yield: 85 % w/w. XRPD of crystalline compound is in accordance with the one reported in Chinese patent CN101412700B.

## Claims

1. A process comprising conversion of formyl group of compound of formula II to cyano group, to produce compound of formula III, in the presence of ammonia, oxygen, and a metal catalyst, wherein the temperature range is 60-120 °C, 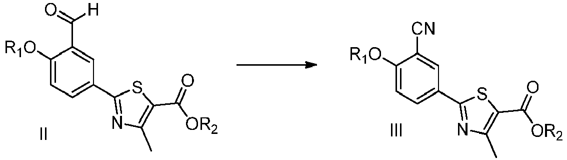 wherein R₁ is a hydrogen atom, an alkyl, alkenyl, or alkynyl group wherein chain group is either straight or branched with 1 to 15 carbon atoms and R₂ is an alkyl, alkenyl, or alkynyl group wherein chain group is either straight or branched with 1 to 15 carbon atoms.

2. A process for the production of Febuxostat comprising the following steps:
a) conversion of formyl group of compound of formula IIb wherein R₁ is an *iso-*butyl group and R₂ is as defined in claim 1 to cyano group, to produce compound of formula IIIb, in the presence of ammonia, oxygen, and a metal catalyst, wherein the temperature range is 60-120 °C, 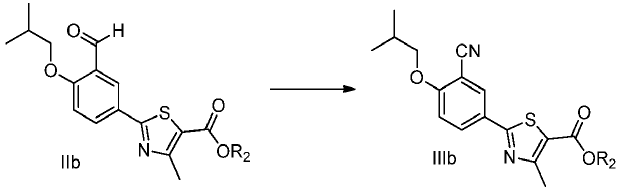
b) hydrolysis of the ester group of compound of formula IIIb to produce Febuxostat, or a salt thereof.

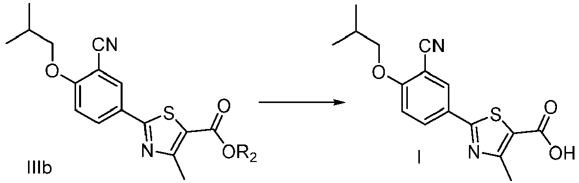

3. A process for the production of Febuxostat comprising the following steps:
a) conversion of formyl group of compound of formula IIa wherein R₁ is a hydrogen atom and R₂ is as defined in claim 1 to cyano group, to produce compound of formula IIIa, in the presence of ammonia, oxygen, and a metal catalyst, wherein the temperature range is 60-120 °C, 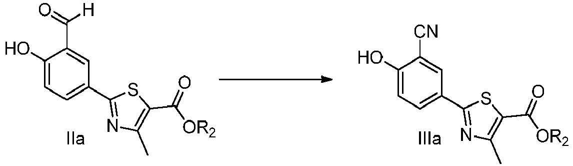
b) alkylation of compound of formula IIIa to compound of formula IIIb wherein wherein R₁ is an *iso*-butyl group and R₂ is as defined in claim 1; 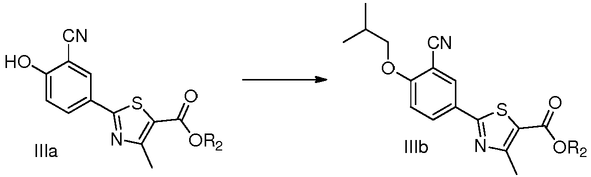
c) hydrolysis of the ester group of compound of formula IIIb to produce Febuxostat, or a salt thereof.

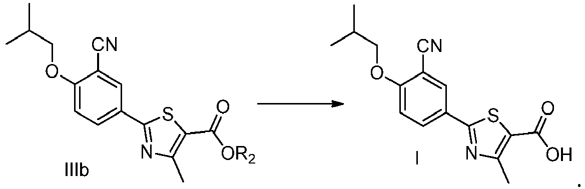

4. A process according to claims 2 or 3 wherein R₂ is an ethyl group.

5. A process, according claims 1-3, wherein the metal catalyst is a copper, iron or ruthenium catalyst.

6. A process, according to claim 5, wherein the metal catalyst is preferably a copper catalyst, preferably copper (I) or (II) halide, copper (I) or (II) nitrate, copper (I) or (II) acetate, copper (I) or (II) sulfate, copper (I) or (II) triflate, copper (I) or (II) oxide and their hydrates.

7. A process, according to claims 1-3, wherein the reaction is performed in a polar aprotic solvent, preferably acetonitrile, dimethylsulfoxide, dimethylformamide, dimethylacetamide, *N*-methyl pyrrolidone or tetrahydrofuran.

8. A process, according to claims 1-3, wherein the reaction is performed under an atmosphere with a composition comprising oxygen in a range 1-100%, under 101,325 KPa pressure to 20265 KPa.

9. A process, according to claim 8, wherein the reaction is performed under normal atmosphere composition and pressure.

## Patentansprüche

1. Verfahren, umfassend die Umwandlung der Formylgruppe der Verbindung der Formel II in eine Cyanogruppe, um die Verbindung der Formel III in Gegenwart von Ammoniak, Sauerstoff und einem Metallkatalysator herzustellen, wobei der Temperaturbereich 60-120°C beträgt, 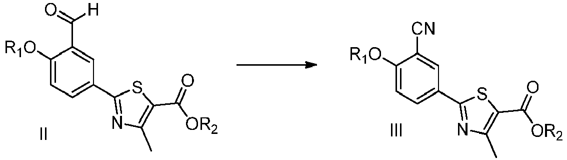 wobei R₁; ein Wasserstoffatom und Alkyl, Alkenyl oder Alkinyl Gruppe, worin die Kettengruppe entweder geradkettig oder verzweigt mit 1 bis 15 Kohlenstoffatomen ist und R₂ eine Alkyl-, Alkenyl- oder Alkinylgruppe ist, worin die Kohlengruppe entweder geradkettig oder verzweigt mit 1 bis 15 Kohlenstoffatomen ist.

2. Verfahren zur Herstellung von Febuxostat, umfassend die folgenden Schritte:
a) Umwandlung einer Formylgruppe der Verbindung der Formel IIb, worin R₁ eine Isobutylgruppe ist und R₂ wie in Anspruch 1 definiert ist, in eine Cyanogruppe, um eine Verbindung der Formel IIIb herzustellen in Gegenwart von Ammoniak, Sauerstoff und einem Metallkatalysator, wobei der Temperaturbereich 60-120°C beträgt, 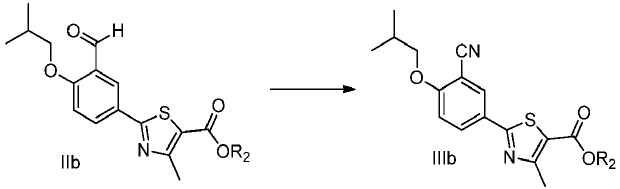
b) Hydrolyse der Estergruppenverbindung der Formel IIIb zur Herstellung von Febuxostat oder eines Salzes davon.

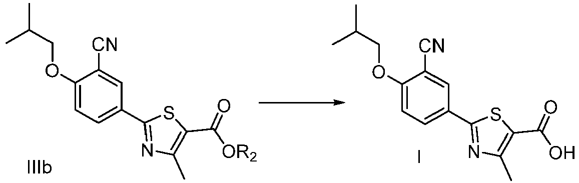

3. Verfahren zur Herstellung von Febuxostat, umfassend die folgenden Schritte:
a) Umwandlung der Formylgruppe der Verbindung der Formel IIa, worin R₁ ein Wasserstoffatom ist und R₂ wie in Anspruch 1 definiert ist, in eine Cyanogruppe, um die Verbindung der Formel IIIa herzustellen, in die Anwesenheit von Ammoniak, Sauerstoff und einem Metallkatalysator, wobei der Temperaturbereich zwischen 60 und 120 °C liegt, 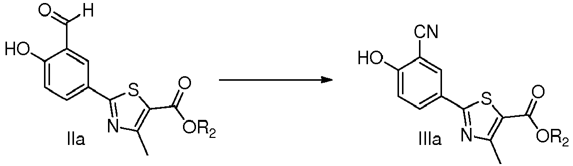
b) Alkylierung der Verbindung der Formel IIIa zur Verbindung der Formel IIIb, wobei R₁ eine Isobutylgruppe ist und R₂ wie in Anspruch 1 definiert ist; 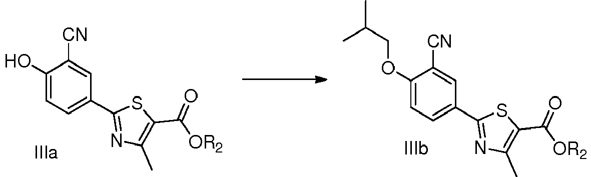
c) Hydrolyse der Estergruppe der Verbindung der Formel IIIb, um Febuxostat oder ein Salz davon herzustellen.

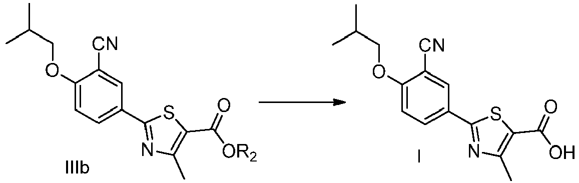

4. Verfahren nach den Ansprüchen 2 oder 3, worin R₂ eine Ethylgruppe ist.

5. Verfahren nach Anspruch 1 bis 3, wobei der Metallkatalysator ein Kupfer-, Eisen- oder Rutheniumkatalysator ist.

6. Verfahren nach Anspruch 5, wobei der Metallkatalysator vorzugsweise ein Kupferkatalysator ist, vorzugsweise Kupfer (I) oder (II) -halogenid, Kupfer (I) oder (II) -nitrat, Kupfer (I) oder (II) -acetat, Kupfer (I) oder (II) Sulfat, Kupfer (I) oder (II) Triflat, Kupfer (I) oder (II) Oxid und deren Hydrate.

7. Verfahren nach den Ansprüchen 1 bis 3, wobei die Reaktion in einem polaren aprotischen Lösungsmittel, vorzugsweise Acetonitril, Dimethylsulfoxid, Dimethylformamid, Dimethylacetamid, *N*-Methylpyrrolidon oder Tetrahydrofuran, durchgeführt wird.

8. Verfahren nach den Ansprüchen 1 bis 3, wobei die Reaktion unter einer Atmosphäre mit einer Zusammensetzung, die Sauerstoff in einem Bereich von 1 bis 100% enthält, unter einem Druck von 101,325 kPa bis 20.265 kPa durchgeführt wird

9. Verfahren nach Anspruch 8, wobei die Reaktion unter normaler Atmosphärenzusammensetzung und normalem Druck durchgeführt wird.

## Revendications

1. Un procédé comprenant la conversion du groupe formyle du composé de formule II en groupe cyano, pour produire le composé de formule III, en présence d'ammoniac, d'oxygène, et d'un catalyseur métallique, et où la plage de température est de 60-120°C, 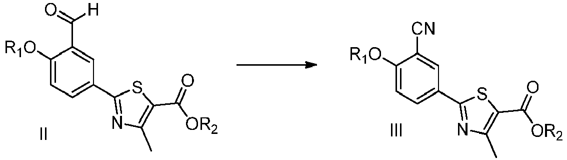 dans lequel, R₁ est un atome d'hydrogène, un alkyle, alcényle ou alcynyle groupe où la chaîne du groupe est soit linéaire ou ramifiée avec de 1 à 15 atomes de carbone et R₂ est un alkyle, alcényle ou alkynyle groupe dans lequel la chaîne du groupe est soit linéaire ou ramifiée avec de 1 à 15 atomes de carbone.

2. Un procédé pour la production du Fébuxostat comprenant les étapes suivantes :
a) conversion du groupe formyle du composé de formule IIb, dans lequel R₁ est un groupe *iso*-butyle et R₂ est tel que défini dans la revendication 1, en groupe cyano pour produire le composé de formule IIIb, en présence d'ammoniac, d'oxygène et d'un catalyseur métallique, et la plage de température est de 60-120°C, 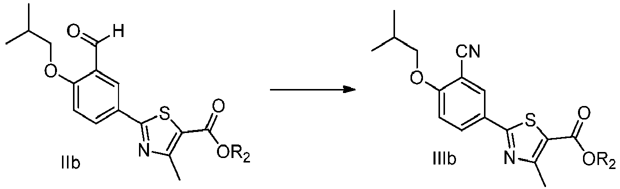
b) hydrolyse du groupe ester du composé de formule IIIb pour produire le Fébuxostat, ou un sel de celui-ci.

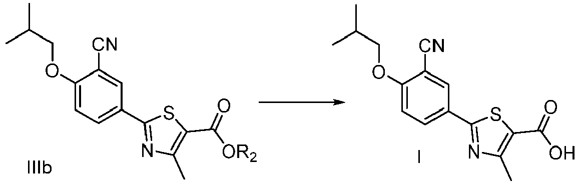

3. Un procédé pour la production du Fébuxostat comprenant les étapes suivantes:
a) conversion du groupe formyle du composé de formule IIa, dans lequel R₁ est un atome d'hydrogène et R₂ est tel que défini à la revendication 1, en groupe cyano pour produire le composé de formule IIIa, en présence d'ammoniac, d'oxygène et d'un catalyseur métallique, et la plage de température est de 60-120°C, 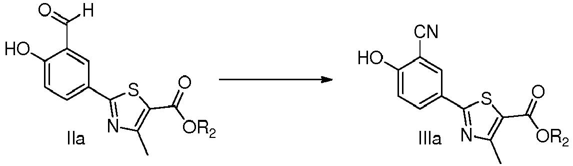
b) alkylation du composé de formule IIIa en composé de formule IIIb, dans lequel R₁ est un groupe *iso*-butyle et R₂ est tel que défini dans la revendication 1, 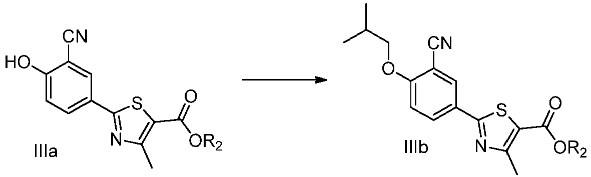
c) hydrolyse du groupe ester du composé de formule IIIb pour produire le Fébuxostat, ou un sel de celui-ci.

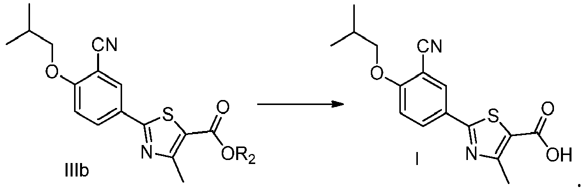

4. Un procédé selon les revendications 2 ou 3, dans lequel R₂ est un groupement éthyle.

5. Un procédé, selon les revendications 1-3, dans lequel le catalyseur métallique est un catalyseur de cuivre, de fer ou de ruthénium.

6. Un procédé, selon la revendication 5, dans lequel le catalyseur métallique est de préférence un catalyseur de cuivre, de préférence un halogénure de cuivre (I) ou (II), un nitrate de cuivre (I) ou (II), un acétate de cuivre (I) ou (II), un sulfate de cuivre (I) ou (II), un triflate de cuivre (I) ou (II), un oxide de cuivre (I) ou (II) et leurs hydrates.

7. Un procédé, selon les revendications 1-3, dans lequel la réaction est réalisée dans un solvant aprotique, de préférence l'acétonitrile, le diméthylsulfoxyde, le diméthylformamide, le diméthylacétamide, la N-méthyl pyrrolidone ou le le tétrahydrofurane.

8. Un procédé, selon les revendications 1-3, dans lequel la réaction est réalisée sous une atmosphère dont la composition comprend de l'oxygène dans une gamme de 1-100%, sous une pression de 101,325 KPa à 20265 KPa.

9. Un procédé, selon la revendication 8, dans lequel la réaction est réalisée sous atmosphère de composition normale et pression normale.
